# FASCICULE DE BREVET EUROPEEN

(11) **EP 2 647 614 B1**
(45) Date de publication et mention de la délivrance du brevet: **08.01.2020**
(21) Numéro de dépôt: 12002508.5
(22) Date de dépôt: 05.04.2012
(51) Int. Cl.: C05F 9/04, C05F 17/00

(54) **Procédé de préparation de complexes végétaux activés et de complexes végétaux/matières organiques dopés ou surdopés, carbonés et leurs applications notamment en méthanisation ou fabrication de biogaz**
Aufbereitungsverfahren von aktivierten Pflanzenkomplexen und dotierten oder überdotierten, kohlenstoffhaltigen Komplexen aus pflanzlichem/organischem Material, und ihre Anwendungen, insbesondere bei der Methanisierung oder der Herstellung von Biogas
Method for preparing activated plant complexes and plant/organic matter complexes that are doped or overdoped, carbonated, and the uses thereof in particular in anaerobic digestion or biogas production

(43) Date de publication de la demande: 09.10.2013
(73) Titulaire: Mezy, Marcel Léon, 12340 Bozouls (FR)
(72) Inventeur: Mezy, Marcel Léon, 12340 Bozouls (FR)

(56) Documents cités:
- EP-A2- 0 356 816
- DE-A1- 3 409 019
- FR-A1- 2 893 612
- LUO J ET AL: "The use of pine bark and natural zeolite as biofilter media to remove animal rendering process odours", BIORESOURCE TECHNOLOGY, ELSEVIER BV, GB, vol. 97, no. 13, 1 septembre 2006 (2006-09-01), pages 1461-1469, XP027965302, ISSN: 0960-8524 [extrait le 2006-09-01]

## Description

### SECTEUR TECHNIQUE

La présente invention concerne le secteur technique des matières organiques et végétales, des biomasses, et des composts de manière générale, de la préparation de composts complexes, de diverses formes de présentation, et de leurs applications notamment en méthanisation.

Il a été découvert selon l'invention :
- un procédé de préparation,
- à partir de complexes végétaux (complexes de plantes diverses CPL) et d'un élément couvrant comme une paille P,
- d'une part a) de ce que nous nommerons ici pour simplifier des « complexes végétaux » CV dont on expliquera qu'ils sont extrêmement activés,
- d'autre part b) de ce que nous nommerons ici par simplicité des « complexes végétaux dopés » ou « surdopés » (R1 ou R2).
- Ces termes font référence aux figures annexées et seront expliqués en détail ci-dessous.

On verra que la présente invention conduit à des complexes végétaux dopés ou surdopés, complexe qui agit dans des milieux très variés aérobies et anaérobies. Il a notamment pour effet :
- d'accélérer de l'action de microorganismes aérobies et anaérobies de 1,1 à 10 fois plus et généralement de 1,3 à 3 fois plus - notamment en compostage et en méthanisation ;
- en conditions anaérobies :
   o d'augmenter la production de biogaz et la proportion de méthane dans ce biogaz, ce qui revient à augmenter la production de méthane de 1,1 à 6 fois plus et généralement de 1,3 à 3 fois plus et de réduire la proportion en CO₂ dans le biogaz de 10 à 90% et généralement de 40 à 60% ;
   o de diminuer les émissions de H2S de 30 à 80% et généralement de 40 à 60% ;
   ∘ d'augmenter la teneur en acides humiques du digestat de méthanisation de 1,1 à 6 fois plus et généralement de 1,3 à 3 fois plus et donc d'améliorer d'autant sa valeur fertilisante ;
- en conditions aérobies :
   ∘ d'accélérer la fixation des éléments de l'air, notamment d'augmenter la fixation de CO₂, d'O₂ et de N₂ de 1,1 à 10 fois plus et généralement de 1,3 à 3 fois plus ;
- en conditions aérobies et/ou anaérobies :
   ∘ d'accélérer la transformation des matières organiques - notamment l'évolution, la décomposition et la synthèse de matières organiques - de 1,1 à 10 fois plus et généralement de 1,3 à 3 fois plus ;
   ∘ de réduire les dégagements/pertes gazeuses et liquides des matières organiques et minérales lors de leur stockage, de leur évolution et de leur épandage, notamment de l'azote et du carbone, de 10 à 90% et généralement de 40 à 60% ;
   ∘ d'accélérer l'extraction et la fixation des éléments organiques et minéraux dans le milieu dans lequel l'invention se trouve, de 1,1 à 10 fois plus et généralement de 1,3 à 3 fois plus, notamment accélérateur de la fixation d'azote et de carbone en créant notamment des « puits de carbone et d'azote » utiles dans la lutte contre le réchauffement climatique et les gaz à effet de serre ;
   ∘ de dépolluer les eaux en réduisant la DCO de 10 à 90% et généralement de 40 à 60% ;
- dans les sols ou le substrat (notamment compost, support de culture, déchet organique à traiter, effluents d'élevage, fumiers, lisiers, ...) dans lequel l'invention est placée en conditions aérobies et/ou anaérobies :
   ∘ d'accélérer la production d'humus et d'acides humiques, de 1,1 à 10 fois plus et généralement de 1,3 à 3 fois plus, avec pour conséquences pour le sol ou le substrat dans lequel l'invention est placée :
      ▪ une augmentation du complexe argilo-humique, notamment de sa taille et de sa CEC (capacité d'échange cationique) de 1,1 à 10 fois plus et généralement de 1,3 à 3 fois plus,
      ▪ une amélioration de sa structure, de sa capacité à se ressuyer en cas d'excès d'eau, de sa facilité de travail, de sa faculté à faciliter le développement racinaire de 1,1 à 10 fois plus et généralement de 1,3 à 3 fois plus,
      ▪ une amélioration de sa capacité de rétention en eau et économie d'eau, notamment d'irrigation et notamment dans un contexte de lutte contre la sécheresse de 1,1 à 10 fois plus et généralement de 1,3 à 3 fois plus,
      ▪ une augmentation de sa résistance à l'érosion de 1,1 à 10 fois plus et généralement de 1,3 à 3 fois plus,
      ▪ une amélioration de sa fertilité, notamment de la quantité d'éléments nutritifs disponibles pour les plantes de 1,1 à 10 fois plus et généralement de 1,3 à 3 fois plus et de l'équilibre des éléments dans les sols ;
   ∘ d'augmenter le développement racinaire de 1,1 à 10 fois plus et généralement de 1,3 à 3 fois plus ;
   ∘ d'augmenter le développement de microorganismes rhizosphériques, notamment de mycorhizes, de 1,1 à 10 fois plus et généralement de 1,3 à 3 fois plus ;
   ∘ d'augmenter la restitution des éléments nutritifs aux plantes de 1,1 à 10 fois plus et généralement de 1,3 à 3 fois plus ;
   ∘ d'augmenter la résistance des plantes aux maladies de 1,1 à 10 fois plus et généralement de 1,3 à 3 fois plus, et donc de réduire l'emploi de produits pesticides de 10 à 100% et généralement de 50 à 100% ;
   ∘ d'augmenter la biodiversité des plantes en prairies comme en cultures multi-espèces de 1,1 à 10 fois plus et généralement de 1,3 à 3 fois plus ;
   ∘ de réduire les mauvaises herbes de 10 à 90% et généralement de 40 à 60% ;
   ∘ d'augmenter la qualité et/ou la quantité des productions agricoles et alimentaires de 1,1 à 10 fois plus et généralement de 1,3 à 3 fois plus, et donc d'améliorer la nutrition qualitative et quantitative des êtres humains de 1,1 à 10 fois plus et généralement de 1,3 à 3 fois plus, dans le contexte d'augmentation de la population mondiale,
   ∘ d'augmenter l'extraction des éléments minéraux inexploités des sols, des sous-sols et des substrats dans laquelle l'invention est placée, de 1,1 à 10 fois plus et généralement de 1,3 à 3 fois plus,
   ∘ d'augmenter la fixation et l'adsorption de métaux lourds, de sels notamment de nitrates ou de tout autre polluant dans un sol ou un substrat contaminé, de 1,1 à 10 fois plus et généralement de 1,3 à 3 fois plus, ce qui a pour conséquence de réduire le transfert de ces polluants aux plantes qui y poussent de 10 à 90% et généralement de 40 à 60%.
   NB : Le terme « matières organiques » est pris dans son sens le plus large, c'est-à-dire qu'il correspond à toute matière contenant du carbone.

Sans vouloir être lié par une quelconque théorie, le Demandeur suggère que les propriétés (dont on mesurera le caractère surprenant et hautement favorable notamment à la méthanisation) sont dues à la combinaison de réactions *soit nouvelles soit fortement « dopées » ou « activées »* entre interfaces solides/liquides/gaz et phases gazeuses et / ou les bactéries , champignons, spores, et autres microorganismes présents (et avec les phases gazeuses de l'atmosphère ambiante) lors de la mise en œuvre du procédé original de l'invention, notamment dégradation initiale des phases végétales, synergie avec des effets de couverture d'une masse par une âtre, fixation des phases gazeuses essentielles comme l'azote ou le CO₂, et *productions corolaires extraordinairement accrues de substances connues ou nouvelles telles que noyaux d'acides humiques et mycorhizes grâce à des échanges multipliés entre phases et percolation lente des phases gazeuses.*

### ART ANTERIEUR et PROBLEMES TECHNIQUES

On connaît naturellement les composts depuis très longtemps. Il s'agit de produits de décomposition plus ou moins avancée de produits naturels (feuilles, herbes, etc...) ou issus de matières d'origine animale (fumier, etc...) ou de mélanges de ces deux types.

Les compositions varient selon le sol d'où il provient, la région, les animaux concernés, etc...

Il s'agit de processus lents, qui du fait de leur lenteur, laissent perdre dans l'environnement (notamment l'air et les eaux) nombre d'éléments, en particulier du carbone et de l'azote, qui se seraient avérés très utiles.

On connaît par ailleurs l'emploi de bactéries pour traiter des eaux résiduaires de tous types.

On sait enfin employer des matières organiques pour produire du gaz méthane (CH₄). Le rendement de l'activité des bactéries produisant de manière bien connue notamment du gaz méthane, est cependant très faible et l'emploi du méthane est donc peu rentable, donc quasi « anecdotique » alors qu'il pourrait devenir une source importante d'énergie. Ici encore, l'invention apporte une amélioration représentant un saut quantique.

Bien que ces problèmes soient bien connus et depuis longtemps, l'industrie concernée n'a trouvé aucune solution technique et donc s'est contentée de développer des palliatifs qui ne règlent pas, et de très loin, la globalité des problèmes.

On citera brièvement la filière agricole, ou un traitement très efficace , écologique et économique de toutes les eaux résiduaires, cf. les propriétés *générales* énoncées ci-dessus, mais surtout **la filière méthane** n'a pas ou très peu évolué, et aucune recherche ne semble à ce jour être de nature à permettre un haut rendement, et aucune recherche intensive n'est menée de ce côté (alors que la production d'énergie dite « verte » ou « propre » a subi un essor récent et très important (éoliennes, panneaux photovoltaïques, etc...), ce qui montre bien que la demande en de telles énergies est importante et n'est pas satisfaite. Le document FR 2 893 612 A1 divulgue un procédé de compostage de déchets organiques en andains comprenant une étape de recouvrement des andains de deux couches, une première couche comprenant du compost mûr et une deuxième couche comprenant des matériaux inorganiques tels que des zéolites, mâchefers, coquillages ou pouzzolane. Ces matériaux possèdent tous des carbonates. Ces couches permettent de limiter les émissions odorantes des andains de compost. Après compostage (c'est à dire quand il n'y a plus de dégagement gazeux de l'andain), le compost est séparé par criblage à l'aide d'un trommel.Le document DE 34 09 019 A1 divulgue un additif pour le compost comprenant des herbes et des algues séchées, ainsi que des carbonate.

### DESCRIPTION GENERALE DE L'INVENTION

« **L'homme de métier** » est dans la présente demande tout homme de l'art travaillant la terre, comme les éleveurs, agriculteurs, sylviculteurs et activités analogues, et possédant une connaissance normale des fermentations, composts, de la « biomasse », et de la production de méthane ou « méthanisation ».

**Le problème technique** posé est de fabriquer du biogaz contenant du méthane, ou du méthane, par un procédé de méthanisation ; avec un rendement en CH₄ très fortement amélioré.

### DESCRITION DETAILLEE DE L'INVENTION et SOLUTION TECHNIQUE

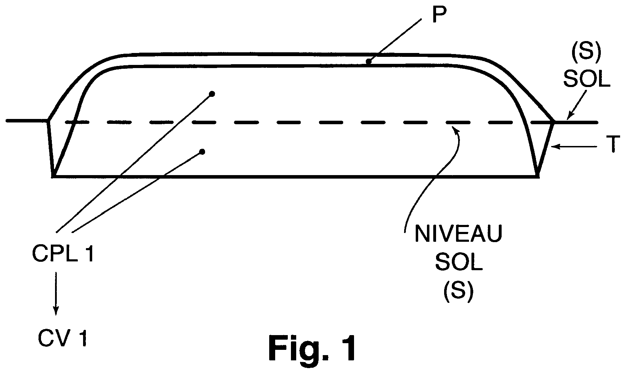

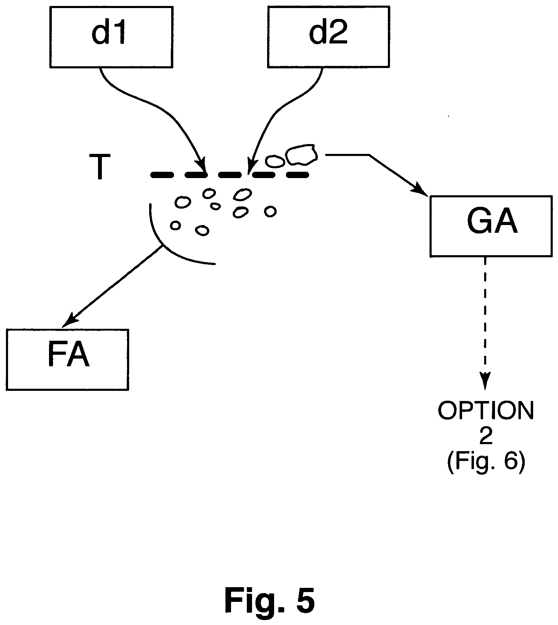

On se réfèrera aux Figures 1 à 6 annexées.

L'invention concerne un procédé de préparation de Complexes végétaux dopés ou surdopés CVD ou CVSD dit « avec couverture d'un compost par au moins un complexe végétal » caractérisé en ce qu'il comporte les étapes suivantes :

### ETAPEA

***Préparation de** « **complexes végétaux (CV) activés »** pouvant différer les uns des autres en fonction des sols et facteurs analogues, ou des mélanges ou associations de matières végétales ou minérales localement présentes et naturelles, et non polluantes ni toxiques.*

**Comme on le verra ces complexes végétaux CV sont déjà « activés » fortement.**

### Etape A1

On récolte un certain nombre, entre 6 et 20 types, de préférence 10 à 15 types, selon les possibilités locales, de matières végétales que l'on appellera ici « complexes de plantes » ou CPL.

Chaque CPL sera constitué *d'une seule plante ou matière végétale particulière* (ou de manière extrêmement prédominante d'une seule plante ou matière végétale particulière, une faible contamination par d'autres matières végétales pouvant être admise car étant souvent inévitable lors du ramassage et du tri).

Ces plantes ou matières *végétales* de préférence (mais à titre non limitatif) comprennent au moins :
- les orties,
- les feuilles de chêne, et
- les champignons, moisissures et spores présents sur le sol, se développant sous les feuilles tombées par exemple, ou dans le sol, sur des racines,
- et optionnellement autres plantes ou matières *végétales* selon les disponibilités locales.

Chaque plante ou matière végétale sera étalée séparément sur une grande surface, par exemple un ha (hectare), éventuellement a demi enfouie dans une tranchée T ménagée dans le sol (S), et on étalera donc ainsi les orties sur une surface, les feuilles sur une autre, les champignons sur une troisième etc...

### ETAPE A2

### 1 Première fraction des ces plantes ou matières végétales : FIG 1

Chaque plante ou matière végétale CPL1 de cette première fraction est étalée sur une grande surface, par exemple un ha, à moitié enfouie dans une tranchée T et à moitié « hors sol ».

Sont particulièrement concernées ici les orties et feuilles de chêne et éventuellement autres plantes.

On a représenté cette première option sur la figure 1 où un CPL1 (complexe de plantes 1, par exemple feuilles de chêne, *OU séparément* orties, etc...) est à demi étalé dans une tranchée T, l'autre moitié (environ) étant hors sol. Le niveau du sol est représenté par (S).

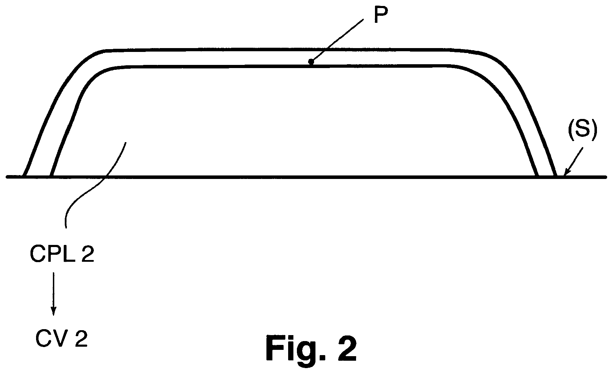

### 2 Seconde fraction des ces plantes ou matières végétales : FIG 2

Chaque plante ou matière végétale CPL2 de cette seconde fraction est étalée sur une grande surface, par exemple un ha, totalement « hors sol »_{.}

Sont particulièrement concernées les feuilles, champignons, et autres plantes.
3 Chacun de ces « étalements » de plantes ou matières végétales est recouvert par une épaisseur de paille P, NON TRAITEE, notamment une paille de blé ou d'épeautre.
4 Chaque « étalement » présente de préférence une épaisseur de l'ordre de 80 cm à 1 m, et l'épaisseur de la couche de paille P est de l'ordre de 20 à 40 cm.

Incidemment, on note que au fil des mois, le « tas » va se tasser du fait de la dégradation des matières végétales.

Au moment de la «récolte» l'épaisseur des plantes sera réduite à autour de 15 à 20 - 30 cm.

### ETAPE A3

5 on laisse l'ensemble des étalements se dégrader durant, à titre d'exemplification efficace, 18 à 24 mois, de préférence 2 ans, pour les étalements semi enfouis de la « première fraction » semi-enfouie, et durant 2 à 8 mois, de préférence 3 à 6 mois, pour les étalements totalement hors sol de la « seconde fraction »_{.}

Après la récolte (cf. ci-dessous) on constate de manière surprenante une très forte dégradation des plantes et matières végétales et une très surprenante croissance de la population de champignons, spores etc... et il semble que cet effet surprenant soit dû à la couverture de paille dont la dégradation influe en synergie sur la dégradation des plantes et matières végétales, avec de plus un effet de couverture qui probablement ralentit fortement les pertes en matières et favorise les échanges.

On juge du moment approprié de la récolte, pour chaque étalement, en fonction du degré de dégradation de la paille recouvrant chaque étalement (aspect, changement de couleur etc...). Ceci fait appel aux connaissances générales de l'homme de métier et à son bon sens.

### ETAPE A4

### 6 Récolte :

Au terme des périodes ci-dessus, on récolte l'ENSEMBLE de la paille dégradée et des plantes et matières végétales dégradées, et de leurs produits de dégradation, dont les champignons, ce qui forme autant de «complexes végétaux» CV que de tas d'étalement, ce qui est représenté par CV1 pour la première fraction et CV2 pour la seconde fraction.

La récolte a lieu de préférence une fois par an, ce qui n'est évidemment pas limitatif.

L'homme de métier comprendra sans difficulté que, les temps de dégradation des première et seconde fractions étant différents, il conviendra de préparer des étalements plus ou moins nombreux et répartis dans le temps selon que la récolte se fera plus ou moins rapidement.

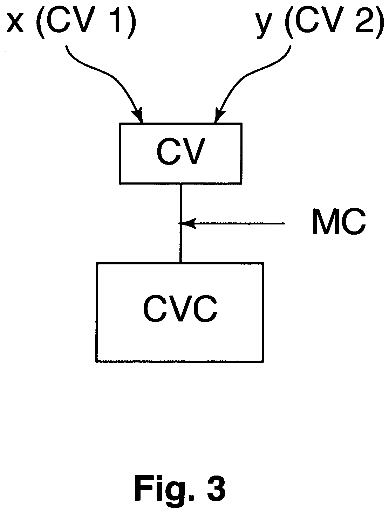

Ces récoltes des complexes végétaux c'est-à-dire xCV1, yCV2, (déjà « activés » du fait d'une dégradation « dopée ») (autant de CVi que d' « étalements couverts de paille ») sont regroupées et brassées ce qui forme le **« Complexe Végétal » CV (global)** (qui est donc très activé et très complexe) (Fig. 3).

Chaque complexe végétal CV i contient les résidus et produits de dégradation de la plante/matière végétale de départ, et de la paille P mise en couverture, ainsi que la microfaune et la microflore qui, soit étaient déjà présentes et se sont très fortement développées, soit sont apparues en forte proportion, avec notamment une multiplication extraordinaire de toutes sortes de champignons, spores, facteur considéré par le Demandeur comme étant l'une des clés des propriétés surprenantes des produits intermédiaires (CV global et CV carbonaté, cf. ci-dessous), et finaux (Complexes végétaux dopés ou (selon l'option de préparation, cf. ci-dessous) « surdopés »).

### ETAPE B

### Préparation de « complexes végétaux carbonatés » (CVC) fortement actifs

### (Fig. 3)

Le Complexe végétal global (activé) CV préparé dans l'étape B est
- séché jusqu'à une concentration en matières sèches d'environ 60 à 80% en poids, et est ensuite
- mélangé à une « **matière carbonate** ».

Cette « matière carbonate » peut être définie comme toute matière comportant une forte ou très forte proportion de carbonate, de manière totalement préférée de **carbonate de calcium,** sous une forme présentant une granulométrie grossière à très grossière : on préférera donc les GCC ou carbonates de calcium naturels broyés, de tout type bien connu dans le domaine dit des « charges minérales », par exemple marbre, calcite, aragonite etc...

La granulométrie sera dans le domaine de 100 à 300 microns, à titre indicatif, voire plus grossière.

L'invention ne recherche absolument pas une granulométrie homogène, et au contraire la granulométrie peut être très hétérogène. De plus, il sera inévitable que la matière carbonate puisse comprendre un faible pourcentage de fines ou ultrafines (inévitables au broyage) et/ou de particules très grossières.

On notera que l'on préférera une granulométrie hétérogène voire très hétérogène afin que l'atmosphère ambiante (cf. « procédé Option 2 » ci-dessous) puisse traverser facilement la couche de carbonate.

Des listes de tels carbonates sont à la disposition de l'homme de métier dans les brevets et documents traitant de broyage et d'additifs de broyage de carbonates, de charges minérales et additifs pour matières plastiques, peintures, papier, sauces de couchage pour papier, etc... et les courbes granulométriques, d50, etc... en sont notoirement connues.

**La proportion efficace préférée de « matière carbonate »** et notamment de carbonate de calcium est de l'ordre de 70 à 95% en poids de matière carbonate sèche (en général, le taux de matières sèches est dans un carbonate de l'ordre de 98% en poids), de préférence 80 à 90%, le complément à 100% étant le Complexe Végétal global à 60 - 80% en poids de matières sèches, soit de 5 à 30%, de préférence de 10 à 20%, de complexe végétal brut.

### PROCEDE DE PREPARATION de COMPLEXES VEGETAUX DOPES ou SURDOPES

### (Fig. 4, 5, 6)

### OPTION 1

### Complexe végétal dopé (CVD)

### Etape 1 A

On prépare un compost à très forte proportion de paille ou matière végétale équivalente, de manière totalement préférée de paille, et d'une matière organique.

On emploiera de préférence, selon le mode à ce jour le plus efficace, et le plus pratique sur site, un compost de paille et de crottin de cheval.

De manière préférée, ce compost contient 90% en poids de paille et 10% en poids de crottin de cheval.

Encore de manière préférée, ce compost contient 20 à 40% de matières sèches en poids, le solde étant de l'eau.

Il sera très nettement préféré de disposer un compost couvrant CVC sur un compost CP différent, par exemple (à titre non limitatif)
- de manière très préférée un compost **végétal** sur un fumier d'élevage bovin ou **chevalin** (équidés)
- ou un compost végétal sur un fumier d'autres animaux d'élevage.

On indique que la proportion de matières animales est au final toujours inférieure à 1% en poids brut dans les matières données aux microorganismes pour se développer.

### ETAPE 1 B

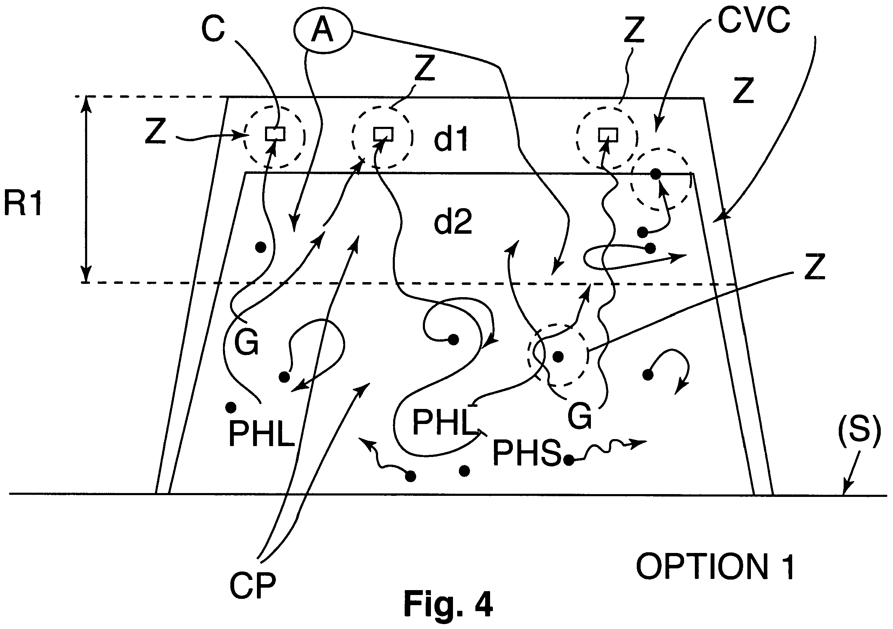

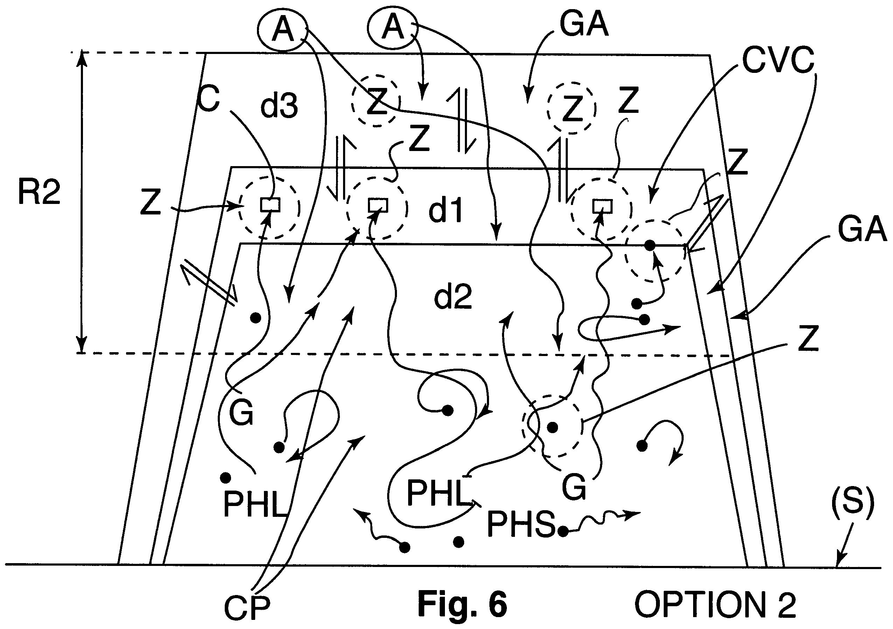

On laisse ce compost fermenter en conditions aérobies durant 3 à 6 jours, ce qui produit le compost fermenté ou en fermentation CP sur les Figures 4 et 6.

### ETAPE 1 C

On dépose sur le sol ce compost CP en forme de tas généralement trapézoïdal en section, et on le recouvre sur toutes ses faces, notamment sa face supérieure, d'une couche de Complexe Végétal Carbonaté CVC obtenu ci-dessus.

La hauteur du tas de compost CP est de l'ordre de 1,70 à 1,90 m.

Sur la face supérieure de ce tas de compost CP , la couche de complexe végétal carbonaté CVC est de l'ordre de 15 à 35 cm, de préférence 20 à 30 cm, et de 5 à 10 cm sur les faces latérales.

### ETAPE 1 D

On laisse le compost CP poursuivre sa fermentation sous la couverture de, et en synergie avec, la couche couvrante de CVC, jusqu'à ce que l'on perçoive au sommet de l'ensemble un dégagement de gaz. Un appareil pour mesurer les gaz dégagés est placé au sommet du tas de compost CP pour déterminer cet état de mûrissement, détermination qui même sans cet appareil est à la portée des connaissances techniques de tout homme du métier, et de son bon sens.

### ETAPE 1 E RECOLTE

Lorsque l'homme de métier estime que le complexe est mûr pour la récolte, on procède à la récolte de : (cf. Fig. 4 Option 1)
- l'ensemble des couches supérieure et latérales de complexe végétal carbonaté CVC (volume de complexe d1) ET d'un volume d2 de compost CP situé sous l'interface entre CVC et CP.

Pour une hauteur de la masse de compost CP de 60 - 80 cm et une hauteur de la couche supérieure de CVC de 15-30, de préférence 20-30 cm, la hauteur du volume d2 de la masse de compost CP prélevé sous l'interface CP/CVC est de l'ordre de 60 à 80 cm.

Bien entendu il se produit des réactions et échanges dans tout le compost inférieur CP donc y compris au dessous du volume d2 qui sera récupéré mais il est évident que ces événements diminuent de plus en plus (selon un effet de gradient) lorsque l'on s'éloigne de ladite interface CVC / CP. On récupère donc le CVC (d1), et un volume d2 du compost CP qui est un **compromis** entre la nécessité de disposer d'un volume récupéré d2 le plus important possible, mais pas trop important de manière à ne pas récupérer un compost d2 trop peu activé car trop loin de l'interface, ce qui dégraderait l'activité globale du complexe d1 + d2 récupéré », ladite activité étant appréciée notamment par une mesure de température et/ou prélèvement d'échantillons pour établir une relation entre température et bioactivité, la bioactivité étant appréciée notamment par analyse des gaz comme CO₂ ou NH₃.

Ce compromis est facilement réalisable par l'homme de métier au besoin avec l'aide de quelques essais de routine, par exemple en effectuant quelques essais sur de petits volumes, comme 100 ou 200 litres, en mesurant la température par exemple par une sonde de température placée à l'interface, en établissant (par prélèvements d'échantillons du compost autour de la zone de la sonde) une relation entre température et bioactivité, ou toute autre méthode simple et pragmatique. De telles méthodes sont connues ou facilement accessibles à tout homme de métier.

Quant au second facteur d'essai possible (avec la température), c'est-à-dire la « bioactivité » du compost spécial prélevé, on peut par exemple facilement mesurer et analyser en continu ou en discontinu des volumes de gaz qui se dégagent ; on pourra par exemple suivre le dégagement de CO₂ ou NH₃ et, comme pour la température, établir une relation simple telle que bioactivité = f (concentration de CO₂ ou NH₃) ou analogue à la portée de tout homme de métier.

On a constaté (ou bien le Demandeur ne s'explique les propriétés et teneurs obtenues que par cette hypothèse raisonnable), pour le produit récolté d1 (CVC) + d2 (CP) :
- une réorganisation des matières végétales et organiques, notamment celles provenant de la paille et du crottin ou fumier ;
- une intervention des gaz de l'atmosphère ambiante (A) comme notamment azote, CO₂, O_{2,} H₂, qui participent aux échanges interphases dans les couches d1 et d2
- l'intervention efficace du carbonate qui participe grandement à la fixation des gaz ammoniac, CO₂, H₂ générés par les échanges interphases ou introduits via l'atmosphère ambiante (A)
- une extrêmement forte concentration en champignons ou de spores, dont une partie a favorisé le fort développement de noyaux d'acides humiques.

Comme indiqué ci-dessus, il semble pour le Demandeur que les propriétés surprenantes obtenues viennent
- de la très forte prolifération des champignons, moisissures et analogues , et des mycorhizes,
- et il semble aussi que la fixation extrêmement importante des gaz comme ammoniac et CO₂ soit essentielle pour former un « puits de carbone et d'azote ».

On note pour l'essentiel des :
- acides humiques, et leurs noyaux
- acides fulviques,
- microorganismes aérobies, notamment champignons, actinomycètes et bactéries aérobies, spores, moisissures, etc...

De plus, les noyaux d'acides humiques et les acides humiques, formés en proportions extrêmement importantes grâce aux champignons, spores etc... contribuent selon le Demandeur à une grande partie essentielle des propriétés.

Par « extrêmement importantes », on indique ici qu'il s'agit de concentrations très largement supérieures à celles auxquelles on pouvait s'attendre au vu des connaissances de l'art antérieur et des pratiques de l'homme du métier, ce qui est évidemment surprenant s'agissant de produits de base manipulés depuis de nombreuses décennies.

Sans vouloir être lié par une théorie, le Demandeur suggère que le fait de « couvrir » un compost CP par une couche de complexe végétal activé et carbonaté CVC va
bloquer ou « fixer » dans une large mesure les dégagements gazeux du compost CP couvert, lesquels dégagements gazeux (CO₂, NH₃, H₂, etc...) vont donc être obligés de « **percoler » lentement** au travers des éléments de fermentation / décomposition du compost CP ; cette percolation doit générer de nombreux échanges interphases et inter-ingrédients, y compris phase gazeuse><phase solide><phase liquide ou pâteuse, qui seront lents et donc extrêmement efficaces. Le carbonate va aider de manière surprenante à fixer ces gaz à la fois ceux de l'atmosphère et ceux du compost CP.
permettre aux bactéries de se développer au maximum et donc de produire des effets dopants maximum (y compris action sur la fermentation, génération plus active de gaz etc...)
permettre des échanges supplémentaires avec la matière carbonate, donner le temps aux gaz atmosphériques (A) d'intervenir dans les couches CVC et d1 de CP et d'y interagir ;
C'est pour bloquer notamment l'échappement des gaz qu'il est nécessaire de **couvrir au maximum,** et si possible en totalité, la surface du compost CP.

La **« matière carbonate » C** employée va intervenir, également selon une théorie du Demandeur, à la fois de par les propriétés connues d'un carbonate (il s'agit d'un produit basique) mais surtout par le fait d'obliger les substances liquides et gazeuses en mouvement à entrer en contact avec des particules grossières:
- offrant une grande surface supplémentaire de contact, ce qui est propice à l'intensification des échanges,
- offrant des possibilités d'adsorption/absorption de certains composants des composts à la surface des particules de carbonate,
- et créant des obstacles et des dédales à la percolation et aux mouvements des phases, offrant ainsi à nouveau des opportunités d'échanges tout en favorisant le brassage des phases.

Ainsi, selon l'invention, et contrairement par exemple à l'évolution aérobie d'un compost de ferme, il ne se produit pas de perte de carbone ou d'azote ou autres éléments utiles, car il n'y a pas perte de gaz de fermentation : au contraire, l'invention emploie ces gaz pour (en combinaison avec les actions bactériennes) participer au dopage et à l'enrichissement en nutritifs et éléments actifs du compost CP notamment dans sa partie récupérable d2, ainsi que du CVC lui même.

On a tenté de schématiser ces phénomènes sur la Fig. 4 annexée, évidemment très succincte.

PHL représente des phases liquides mises en mouvement par convection naturelle ou thermique (chaleur de fermentation)
PHS (points noirs) représente des phases solides moins mobiles.

G représente les gaz cités (O₂, NH₃, H₂, CO₂) qui vont percoler très lentement au travers des masses CP et d2, ralentis par la couverture CVC et par les particules de carbonate C (carrés blancs) qui possèdent leurs propres propriétés d'ad/absorption etc...

Z et les cercles en pointillés représentent quelques unes des zones d'échanges interphases qui en fait apparaissent partout dans les masses, entre de multiples combinaisons de phases impossibles à représenter.

On voit que les gaz de l'atmosphère A peuvent pénétrer dans la couche CVD d1 et au moins d2 et intervenir : comme les produits selon l'invention, entre autres propriétés surprenantes, « fixent » ces gaz, notamment N2 et CO₂ (carbone), cet apport est important.

Les traits fléchés représentent schématiquement les mouvements possibles, percolation, convection, mouvements Browniens etc....

### ETAPE 1 F Passage au Crible ou Tamis T

### (Fig. 5)

Le « Complexe végétal dopé » CVD (en fait il s'agit d'un complexe de matières végétales dégradées dopées par un compost fermenté de paille et de crottin de cheval, dans le mode de réalisation préféré, donc comportant une fraction de matières organiques) récolté, c'est-à-dire le volume d1 (CVD) et d2 (compost CP) est passé sur un crible ou tamis T qui retient les particules de tailles comprises entre environ 1 et 10 mm, ce qui forme un premier produit de « particules grossières activées » ou « GA », et laisse passer les particules de tailles inférieures à sensiblement 1 mm, dénommées ici particules « fines activées » ou « FA ».

Les particules les plus fines sont employées **telles quelles ou diluées** dans les applications envisagées. Cf. section « dilution » ci-dessous.

Quant aux particules grossières activées GA qui représentent selon la théorie du Demandeur un élément important de l'invention, elles seront employées dans le même procédé que ci-dessus, mais à titre de couche supérieure supplémentaire, pour produire un « Complexe Végétal SurDopé » (contenant comme ci-dessus une fraction de dopage par une fraction de matière organique).

### OPTION 2

### Complexe Végétal SurDopé (CVSD)

On met en œuvre strictement les mêmes étapes que dans l'option 1 sauf en ce que, après avoir recouvert le compost CP par la couche de CVC (complexe végétal carbonaté), on recouvre le tout par une troisième couche composée de particules grossières activées GA obtenues par refus au tamis dans l'option 1 ci-dessus.

### Cf. Fig. 6.

Les phénomènes et échanges interphases sont les mêmes, sauf en ce que le complexe CVD est activé « en sandwich » à la fois par le compost couvert CP et par la couche de particules grossières activées GA (laquelle laisse pénétrer les gaz de l'atmosphère A de par sa morosité naturelle).

L'un des intérêts de la couche GA, outre celui de réaliser encore un ralentissement supplémentaire de la percolation, et donc une encore meilleure fixation des gaz et production d'acides humiques, est d'apporter au système réactif des noyaux d'acides humiques et une forte concentration de champignons. D'où la notion d'activation « en sandwich ».

Les effets, et les produits obtenus, seront essentiellement les mêmes que dans l'option 1, sauf en ce que le « dopage » sera plus prononcé, d'environ 10 à 20%, et en ce que l'on aura utilement réutilisé les particules GA difficiles autrement à employer sur site.

Les phénomènes d'échanges sont représentés sur la Fig. 6 annexée. Ce sont les mêmes que ceux expliqués en relation avec la Fig. 4 annexée (option 1) sauf en ce que la couche GA autorise des échanges supplémentaires (on rappelle que à la fois CP et GA sont activés fortement) représentés schématiquement par des double flèches.

On récupère d1 de CVD et d2 de CP, comme dans l'option 1, plus la couche (en général de 40 - 50 - 60 cm au sommet) GA, le rapport entre l'ensemble de ce qui est récolté (d1 + d2 + GA) / reste inutilisé du CP étant alors de l'ordre de 50/50 en volume.

Après criblage, on obtient un Complexe Végétal SurDopé utilisable tel quel ou dilué (cf. section « dilution » ci-dessous).

En fait l'un des mérites du Demandeur est d'avoir songé à bloquer ou ralentir les pertes de gaz lors de la fermentation, et cela en combinant trois composts, dont un était en fermentation aérobie et est ensuite couvert par un compost carbonaté et par un compost grossier

L'invention réalise donc une combinaison de blocage des gaz, dont l'hydrogène, et de fixation des autres gaz dont CO₂, dans la masse : d'où absence de pertes en carbone, azote et autres éléments utiles, c'est-à-dire la création d'un « puits de carbone et d'azote ».

### - PRODUITS INDUSTRIELS NOUVEAUX

Il résulte donc des deux Options de procédé deux « **produits industriels nouveaux** » CVD et CVSD, de par leur « bioactivité » et leur composition, dont la nouveauté peut être (cf. ci-dessous) établie par des « marqueurs » comme :
- le fait que les gaz NH₃, CO₂, aussi H₂, ainsi « bloqués » peuvent alors être fixés par, probablement, action de l'hydrogène qui est lui aussi bloqué. Ainsi le Demandeur a noté que, selon l'invention, 1 t de fumier 2 composté selon l'invention donnait 840 kg de produit restant, tandis qu'un fumier aérobie normal de ferme, par exemple, ne laissait subsister, après le même laps de temps, 6 mois, que 600 kg. On mesure donc la surconcentration opérée par l'invention, et la conservation d'éléments utiles (carbone, azote) qui disparaissent (souvent sous forme toxique de CO₂ ou ammoniac) dans un fumier normal.
- La concentration en noyaux d'acides humiques et acides humiques (de 200 à 600% (notamment 300 - 500%)) supérieure à la concentration attendue par l'homme de métier sans mise en œuvre du procédé à « couverture » de l'invention.
- La concentration et la diversité de microorganismes (bactéries, champignons, moisissures, ...) qui vont servir à fabriquer à leur tour des acides humiques
- Une concentration exceptionnelle en mycorhizes qui sont des champignons qui se fixent sur les racines et sont capables de rechercher l'eau à une profondeur de 5 à 10 fois plus importante que les racines, au bénéfice des plantes.

Ce produit peut aussi être éventuellement caractérisé et différencié des composts habituels grâce à un « marqueur » intermédiaire qui est le taux d'hémicellulose dans le complexe CVD ou CVSD ; ce taux est en effet environ le double dans un produit selon l'invention et prêt à l'emploi et ayant été récupéré au terme de 6 à 8 mois d'interactions par rapport à un compost habituel.

### Dilution : Complexe CVD ou CVSD selon l'invention et ses différentes variantes de présentation :

**CVD ou CVSD « pur »** : les produits purs sont extrêmement actifs et concentrés en matières actives dont les noyaux d'acides humiques, avec fixation de très fortes doses (très surprenantes) de carbone et d'azote.

Ce produit pur peut être employé tel quel, c'est-à-dire sans dilution. Cependant, il est évidemment très concentré et l'emploi d'un produit aussi concentré peut poser des problèmes pratiques (équipement inadapté , risque de surdosage, etc...)

### Dilution avec un support « neutre » ou « inerte »

Le complexe pur CVD ou CVSD (*indifféremment*) selon l'invention peut être dilué avec un ou plusieurs supports solides inertes ou neutres, comme du sable de carbonate ou de silice, ou d'autres produits comme les pouzzolanes, etc... La dilution peut être effectuée dans une proportion à peu près quelconque, qui sera du jugement de l'utilisateur final, notamment dans un rapport CVD ou CVSD pur/sable (en poids brut) de 30/70, 20/80, voire même 10/90, 5/95, 1/99, tant le produit est actif et efficace.

### Dilution avec un support de type « matières organiques » (ou contenant de telles matières organiques)

La dilution peut être effectuée avec toutes sortes de produits contenant un pourcentage de matières organiques connues, comme de tourteaux de colza, de la pulpe d'olives, des cabosses de cacao etc...

L'invention couvre ces produits industriels nouveaux, purs ou dilués et de manière générale les produits industriels nouveaux caractérisés en ce qu'ils consistent en ou comprennent au moins un CVD ou CVSD ou leurs mélanges , purs et/ou dilués.

Le produit peut être utilisé seul à raison de 200 à 500 g/t de fumier. Dans un deuxième cas, il peut être mélangé à une matière minérale (produit qui va dans le fumier avec 5 à 20% de noyau) et ce produit sera positionné à la dose de 1 à 5 Kg/t de fumier.

Le produit qui sera positionné dans les champs peut être mis quand il est pur de 20 à 40 Kg/Ha. Comme il est très difficile de le répartir de manière homogène à une telle dose, il est additionné de matières minérales et organiques de 5 à 10% de noyau et est positionné sur les sols à raison de 200 à 600 Kg/Ha.

Il a maintenant été découvert de manière surprenante une application totalement inattendue de ces produits pour produire du biogaz contenant du méthane, ou du méthane, avec un rendement allant de « amélioré » (lorsque les matières organiques de départ sont peu riches) à « extrêmement augmenté » lorsque les matières organiques de départ sont normales à excellentes.

### METHANISATION / PRODUCTION DE METHANE / production de biogaz contenant du méthane

On connaît des « méthaniseurs » classiques : on fait fermenter dans des fermenteurs ouverts ou fermés, par exemple 3000 m³ de déchets organiques divers tels que : boues de station d'épuration, résidus industriels à composante organique comme les graisses, déchets alimentaires, lisier, fumiers divers, avec évidemment une concentration maximale en matières organiques, que l'on complémente, dans cet exemple de volume, chaque jour, par environ 60 t des mêmes types de déchets organiques, et l'on produit ainsi chaque jour un biogaz contenant notamment, entre autres, du méthane CH₄ (entre 3 et 5 tonnes).

Si, selon l'invention, à ce complément journalier de 60 t de déchets organiques, on ajoute et mélange seulement 600 kg du CVD ou CVSD pur selon l'invention dilué dans les proportions indiquées ci-dessus avec du sable, le rendement en CH₄ est multiplié par 120 à 200 - 220 - 350%, en fonction des déchets organiques de départ. Même un gain « faible » de 20% représente une avancée certaine. Si l'on travaille avec des milieux organiques riches comme des boues d'épuration, on atteint les valeurs les plus élevées. On note également une odeur de H2S bien moins forte.

L'utilisation du produit selon l'invention dans des méthaniseurs pour produire du méthane ou du biogaz permet d'augmenter la production de biogaz et la proportion de méthane dans ce biogaz, ce qui revient à augmenter la production de méthane de 1,1 à 6 fois plus et généralement de 1,3 à 3 fois plus et de réduire la proportion en CO₂ dans le biogaz de 10 à 90% et généralement de 40 à 60%.

De plus, il reste dans le réacteur de méthanisation un fond de réacteur dénommé « **digestat** ». Il s'agit d'un produit sous forme de boue semi-liquide.

Lorsque le produit selon l'invention a été employé pour la production de méthane, ce digestat présente des propriétés extrêmement intéressantes.

Il contient environ le double ou triple d'acides humiques (notamment de 140 à 200 % de plus) et, de plus, élément très important, les matières organiques sont « organisées » en produits assimilables, tandis que l'azote est beaucoup mieux fixée par notamment les acides humiques.

Selon une théorie non limitative, le Demandeur considère que les acides humiques peuvent floculer avec l'argile du sol lors de l'épandage du digestat, ces floculats s'intercalant entre les plaquettes d'argile ce qui va contribuer à aérer le sol et à permettre aux racines de s'insérer dans l'argile jusqu'au contact des acides, où se trouve précisément fixé l'azote nutritif.

Pour l'application « méthanisation », il conviendra d'employer de manière très préférée un sable de carbonate, en proportion de 35 à 60% en poids ramené à CaO, de préférence de 45 à 54% en poids par rapport au produit compost spécial pur de l'invention.

Ce gaz peut être utilisé dans toutes ses applications connues.

Le digestat récupéré est caractérisé en ce qu'il contient environ le double ou triple d'acides humiques (notamment de 140 à 200% de plus) par rapports aux digestats classiques sans emploi de CVD ou CVSD.

## Revendications

1. Procédé de préparation de Complexes végétaux dopés ou surdopés CVD ou CVSD « à couverture d'un compost » , **caractérisé en ce qu'**il comporte les étapes suivantes :
***ETAPEA***
***Préparation de « complexes végétaux (CV) activés***
Etape A1
On récolte un certain nombre, entre 6 et 20 types, de préférence 10 à 15 types, de matières végétales, dits « complexes de plantes » ou CPL, chaque CPL étant constitué *d'une seule plante ou matière végétale particulière,* plantes ou matières *végétales* qui comprennent au moins i :
- les orties,
- les feuilles de chêne, et
- les champignons, moisissures et spores présents sur le sol, se développant sous les feuilles tombées par exemple, ou dans le sol, sur des racines,
- et optionnellement autres plantes ou matières *végétales* selon les disponibilités locales.
et chaque plante ou matière végétale est étalée séparément sur une grande surface, par exemple un ha (hectare), éventuellement à demi enfouie dans une tranchée T ménagée dans le sol (S).
ETAPE A2
1 *Première fraction des ces plantes ou matières végétales :*
Chaque plante ou matière végétale CPL1 de cette première fraction est étalée sur une grande surface, par exemple un ha, à moitié enfouie dans une tranchée T et à moitié « hors sol » notamment orties et feuilles de chêne et éventuellement autres plantes ;
2 *Seconde fraction de ces plantes ou matières végétales :*
Chaque plante ou matière végétale CPL2 de cette seconde fraction est étalée sur une grande surface, par exemple un ha, totalement « hors sol » notamment les feuilles, champignons, et autres plantes ;
3 Chacun de ces « étalements » de plante ou matière végétale est recouvert par une épaisseur de paille P, NON TRAITEE, notamment une paille de blé ou d'épeautre ;
4 Chaque « étalement » présente de préférence une épaisseur de l'ordre de 80 cm à 1 m, et l'épaisseur de la couche de paille P est de l'ordre de 20 à 40 cm ;
ETAPE A3
5 on laisse l'ensemble des étalements se dégrader durant 18 à 24 mois, de préférence 2 ans, pour les étalements semi-enfouis de la « première fraction » semi-enfouie, et durant 2 à 8 mois, de préférence 3 à 6 mois, pour les étalements totalement hors sol de la « seconde fraction » ;
ETAPE A4
6 Récolte :
Au terme des périodes ci-dessus, on récolte l'ENSEMBLE de la paille dégradée et des plantes et matières végétales dégradées, et de leurs produits de dégradation, dont les champignons, ce qui forme autant de « complexes végétaux » CV que de tas d'étalement, et ces récoltes des complexes végétaux c'est-à-dire autant de CVi que d' « étalements couverts de paille ») sont regroupés et brassés ce qui forme le **« Complexe Végétal » CV (global) ;**
***ETAPE B***
***Préparation de** « **complexes végétaux carbonatés » (CVC) fortement actifs***
Le Complexe végétal global (activé) CV préparé dans l'étape B est
- B1 séché jusqu'à une concentration en matières sèches d'environ 60 à 80% en poids, et est ensuite
- B2 mélangé à une « **matière carbonate** » qui est toute matière comportant une forte ou très forte proportion de carbonate, de manière totalement préférées de **carbonate de calcium,** sous une forme présentant une granulométrie grossière à très grossière notamment GCC ou carbonates de calcium naturels broyés, par exemple marbre, calcite, aragonite et de granulométrie dans le domaine de 100 à 300 microns, voire plus grossière, avec de préférence une granulométrie hétérogène voire très hétérogène, la proportion de « matière carbonate » et notamment de carbonate de calcium étant de 70 à 95% en poids de matière carbonate sèche (le taux de matières sèches est dans un carbonate est de l'ordre de 98% en poids), de préférence 80 à 90%, le complément à 100% étant le Complexe Végétal global à 60-80% en poids de matières sèches, soit de 5 à 30%, de préférence de 10 à 20%, de complexe végétal brut.
***PROCEDE DE PREPARATION de COMPLEXES VEGETAUX DOPES ou SURDOPES***
**OPTION 1**
**Complexe végétal dopé (CVD)**
Etape 1 A
On prépare un compost à très forte proportion de paille ou matière végétale équivalente, de manière totalement préférée une paille, et d'une matière organique notamment un compost de paille et de crottin de cheval qui contient de préférence 90% en poids de paille et 10% en poids de crottin de cheval et qui préférentiellement contient 20 à 40% de matières sèches en poids, le solde étant de l'eau.On dispose un compost CVC sur un compost CP différent, par exemple :
- un compost **végétal** sur un fumier d'élevage bovin ou **chevalin** (équidés)
- ou un compost **végétal** sur un fumier d'autres animaux d'élevage,
la proportion de matières animales dans le CP étant au final toujours inférieure à 1% en poids brut dans les matières données aux microorganismes pour se développer.
ETAPE 1 B
On laisse ce compost fermenter en conditions aérobies durant 3 à 6 jours, ce qui produit le compost fermenté ou en fermentation CP.
ETAPE 1 C
On dépose sur le sol ce compost CP en forme de tas de préférence généralement trapézoïdal en section, et on le recouvre sur toutes ses faces, notamment sa face supérieure, d'une couche de Complexe Végétal Carbonaté CVC obtenu ci-dessus.
La hauteur du tas de compost CP est de l'ordre de 1,70 à 1,90 m.
Sur la face supérieure de ce tas de compost CP, la couche de complexe végétal carbonaté CVC est de l'ordre de 15 à 35, de préférence 20 à 30 cm, et de 5 à 10 cm sur les faces latérales.
ETAPE 1 D
On laisse le compost CP poursuivre sa fermentation sous la couverture de, et en synergie avec, la couche couvrante de CVC, jusqu'à ce que l'on perçoive au sommet de l'ensemble un dégagement de gaz.
ETAPE 1 E RECOLTE
On procède alors à la récolte de :
- l'ensemble des couches supérieure et latérales de complexe végétal carbonaté CVC (volume de complexe d1) ET d'un volume d2 de compost CP situé sous l'interface entre CVC et CP.
Pour une hauteur de la masse de compost CP de 60 - 80 cm et une hauteur de la couche supérieure de CVC de 15-30, de préférence 20-30 cm, la hauteur du volume d2 de la masse de compost CP prélevé sous l'interface CP/CVC est de l'ordre de 60 à 80 cm, **compromis** entre la nécessité de disposer d'un volume récupéré d2 le plus important possible, mais pas trop important de manière à ne pas récupérer un compost d2 trop peu activé, ladite activité étant appréciée notamment par une mesure de température et/ou prélèvement d'échantillons pour établir une relation entre température et bioactivité, la bioactivité étant appréciée notamment par analyse des gaz comme CO₂ ou NH₃.
ETAPE 1 F Passage au Crible ou Tamis T
Le « Complexe végétal dopé » CVD récolté, c'est-à-dire le volume d1 (CVD) et d2 (compost CP) est passé sur un crible ou tamis T qui retient les particules de tailles comprises entre environ 1 et 10 mm, ce qui forme un premier produit de « particules grossières activées » ou « GA », et laisse passer les particules de tailles inférieures à sensiblement 1 mm, dénommées ici particules « fines activées » ou « FA » qui forment le Complexe Végétal Dopé CVD.
**OPTION 2**
**Complexe Végétal SurDopé (CVSD)**
On met en œuvres strictement les mêmes étapes que dans l'option 1, sauf **en ce que**, après avoir recouvert le compost CP par la couche de CVC (complexe végétal carbonaté), on recouvre le tout par une troisième couche composée de particules grossières activées GA obtenues par refus au tamis dans l'option 1 ci-dessus.
On récupère d1 de CVD et d2 de CP, comme dans l'option 1, plus la couche (en général de 40 - 50 - 60 cm au sommet) GA, le rapport entre l'ensemble de ce qui est récolté (d1 + d2 + GA) / reste inutilisé du CP étant alors de l'ordre de 50/50 en volume.
Après criblage, on obtient le « Complexe Végétal SurDopé » CVSD.

2. Procédé selon la revendication 1 **caractérisé en ce que** l'on procède à une dilution
- **Dilution avec un support « neutre » ou « inerte »**
Le complexe pur CVD ou CVSD (*indifféremment*) selon l'invention est dilué avec un ou plusieurs supports solides inertes ou neutres, comme du sable de carbonate ou de silice, ou d'autres produits comme les pouzzolanes.
La dilution peut être effectuée dans une proportion à peu près quelconque, notamment dans un rapport CVD ou CVSD pur/sable (en poids brut) de 30/70, 20/80, voire même 10/90, 5/95, 1/99.
- **Dilution avec un support de type « matières organiques » (ou contenant de telles matières organiques)**
La dilution peut être effectuée avec des produits contenant un % de matières organiques comme des tourteaux de colza, de la pulpe d'olives, des cabosses de cacao .

3. Produits CVD et CVSD **caractérisés en ce qu'**ils ont été obtenus par le procédé selon l'une quelconque des revendications précédentes.

4. Produits selon la revendication 3 **caractérisés par** au moins l'une des caractéristiques suivantes ;
- les gaz NH₃, CO₂, aussi H₂ ont été « bloqués » et fixés, ce qui représente un puits de carbone et d'azote ;
- la concentration en noyaux d'acides humiques et acides humiques est de 200 à 600% (notamment 300 - 500%) supérieure à la concentration attendue
- la très grande concentration et la très grande diversité de microorganismes (bactéries, champignons, moisissures,...) pour fabriquer à leur tour des acides humiques
- une concentration exceptionnelle en mycorhizes un taux d'hémicellulose dans le complexe CVD ou CVSD d'environ le double au terme de 6 à 8 mois d'interactions par rapport à un compost habituel.

5. Utilisation d'au moins un produit CVD ou CVSD selon les revendications 3 ou 4 , ou tels que décrits dans les revendications 1 ou 2, purs ou dilués, dans un méthaniseur pour produire du méthane ou biogaz.

6. Utilisation selon la revendication 5 **caractérisée en ce que**, pour un méthaniseur de 3000 m³ employant des déchets organiques, et un complément journalier de 60 t de ces déchets organiques, on ajoute et mélange à ces 60 t de complément journalier 600 kg du CVD ou CVSD pur selon l'invention qui a été dilué dans les proportions indiquées avec du sable et de manière très préférée un sable de carbonate, en proportion de 35 à 60% en poids ramené à CaO, de préférence de 45 à 54% en poids par rapport au produit CVD ou CVSD pur.

7. Utilisation selon la revendication 5 ou 6 **caractérisée en ce que** l'on récupère un fond de réacteur dénommé « **digestat** ».

## Patentansprüche

1. Aufbereitungsverfahren von dotierten oder überdotierten Pflanzenkomplexen DPK oder ÜDPK "zur Abdeckung eines Komposts", **gekennzeichnet durch** die folgenden Etappen:
***ETAPPE A***
***Aufbereitung der" aktivierten Pflanzenkomplexe (PK)"***
Etappe A1
Es wird eine gewisse Anzahl, zwischen 6 und 20 Arten, vorzugsweise 10 bis 15 pflanzliche Materialien, "Pflanzenkomplexe » oder PK genannt, geerntet, jeder PK besteht aus *einer einzigen Pflanze oder aus besonderem pflanzlichen Material,* Pflanzen oder *pflanzlichen* Materialien, die mindestens enthalten :
i :
- Brennnesseln,
- Eichenblätter, und
- die Pilze, Schimmel und Sporen, die im Boden vorhanden sind und sich z. Bsp. unter den gefallenen Blättern oder im Boden auf den Wurzeln entwickeln,
- und gegebenenfalls andere Pflanzen oder *pflanzliche* Materialenjenach den örtlichen Verfügbarkeiten.
und jede Pflanze oder pflanzliches Material wird getrennt auf eine grosse Oberfläche verteilt, zum Beispiel ein ha (Hektar), eventuell halb vergraben
in einen im Boden (B) angelegten Graben (G).
ETAPPE A 2
1 *Erste Fraktion dieser Pflanzen oder pflanzlichen Materialien:*
Jede Pflanze oder pflanzliches Material PK1 dieser ersten Fraktion wird auf eine grosse Oberfläche verteilt, zum Beispiel auf einen Hektar, zur Hälfte eingegraben in einen Graben G und zur Hälfte "hors-sol", vor allem die Brennnesseln und die Eichenblätter und eventuell andere Pflanzen;
2 *Zweite Fraktion dieser Pflanzen oder pflanzlichen Materialien:*
Jede Pflanze oder pflanzliches Material PK2 dieser zweiten Fraktion wird auf eine grosse Oberfläche verteilt, zum Beispiel auf einen Hektar, völlig "hors sol", vor allem die Blätter, Pilze und andere Pflanzen ;
3 Jede dieser "Verteilungen" von Pflanzen oder pflanzlichem Material wird mit einer NICHT BEHANDELTEN Strohschicht S abgedeckt, vor allem mit Weizenstroh oder Dinkelstroh;
4 Jede "Verteilung" hat möglichst eine Dicke von 80 cm bis 1 m, und die Dicke der Strohschicht S beträgt 20 bis 40 cm ;
ETAPPE A3
5 Man lässt die gesamten Verteilungen sich während einer Zeit zwischen 18 bis 24 Monaten zersetzen, vorzugsweise 2 Jahre für die halbeingegrabenen Verteilungen der ersten halbeingegrabenen Fraktion und während 2 bis 8 Monaten, vorzugsweise 3 bis 6 Monate für die vollständig "hors-sol"-Verteilungen der "zweiten Fraktion";
ETAPPE A4
6 Ernte:
Am Ende der oben genannten Perioden werden das GESAMTE zersetzte Stroh und die GESAMTEN zersetzten Pflanzen und pflanzlichen Materialien und deren Zersetzungsprodukte, darunter die Pilze, geerntet, was genau soviele "Pflanzenkomplexe" PK bildet wie Verteilungshaufen, und diese Ernten von Pflanzenkomplexen, das heisst ebenso viele PKi wie "mit Stroh überdeckte Verteilungen", werden zusammengelegt und gemischt, es entsteht der **"Pflanzenkomplex" PK (global);**
***ETAPPE B***
***Aufbereitungsverfahren von hoch aktiven "kohlensäurehaftigen Pflanzenkomplexen (KPK)"***
Der globale Pflanzenkomplex (aktiviert), PK in Etappe B vorbereitet, ist
- B1 getrocknet bis zu einer Konzentration von einer Trockenmasse von etwa 60 bis 80 % des Gewichts, und ist danach
- B2 mit einem "**Karbonatmaterial**" vermischt, das heisst mit jeglichem Material, das einen grossen oder sehr grossen Anteil an Karbonat besitzt, vorzugsweise mit **Kalziumkarbonat,** mit grobkörniger bis sehr grobkörniger Korngrösse, vor allem GCC oder natürliche gemahlene Kalziumkarbonate, wie zum Beispiel Marmor, Kalzit, Aragonit und mit einer Korngrösse im Bereich 100 bis 300
Mikron, oder gröber mit vorzugsweise einer heterogenen, beziehungsweise sehr heterogenen Korngrösse, der Anteil des "Karbonatmaterials" und vor allem des Kalziumkarbonats beträgt zwischen 70 und 95% des Gewichts des trockenen Karbonatmaterials (der Anteil an Trockenmasse beträgt in einem Karbonat 98% des Gewichts), vorzugsweise 80 bis 90%, der Rest für 100% ist der globale Pflanzenkomplex mit 60-80% des Gewichts der Trockenmasse, das heisst zwischen 5 bis 30%, vorzugsweise zwischen 10 bis 20% des Pflanzenkomplexes brutto.
***AUFBEREITUNGSVERFAHREN DER DOTIERTEN UND OBERDOTIERTEN PFLANZENKOMPLEXE***
**OPTION 1**
**Dotierter Pflanzenkomplex (DPK)**
Etappe 1 A
Man bereitet einen Kompost mit einem sehr grossen Anteil an Stroh oder vergleichbarem Pflanzenkomplex vor, insbesondere vorzugsweise Stroh, und mit einer organischen Substanz, vor allem einem Kompost aus Stroh und Pferdemist, der vor allem 90% an Strohgewicht hat und 10% an Pferdemistgewicht, und der vorzugsweise 20 bis 40% an Trockenmassengewicht hat, der Rest besteht aus Wasser. Man legt einen Kompost KPK auf einen anderen Kompost PK, zum Beispiel:
einen Pflanzenkompost auf einen Kuhmist oder **Pferde**mist (Equiden)
oder einen Pflanzenkompost auf den Mist anderer Zuchttiere, Der Anteil des Tiermaterials im PK ist schliesslich immer geringer als 1% des Bruttogewichts in den Stoffen, die den Mikroorganismen für ihre Entwicklung gegeben wurden.
ETAPPE 1 B
Man lässt diesen Kompost unter aeroben Bedingungen während 3 bis 6 Tagen fermentieren, es entsteht der fermentierte Kompost oder ein in Fermentierung stehender PK.
ETAPPE 1 C
Dieser Kompost PK wird in Form eines Haufens auf den Boden abgelegt, allgemein vorzüglich in Trapezform und in Abschnitten, und er wird auf allen Seiten, vor allem auf der oberen Seite, mit einer oben erhaltenen Schicht vom kohlensäurehaltigen Pflanzenkomplex KPK abgedeckt.
Die Höhe des Komposthaufens PK ist zwischen 1,70 und 1,90 m.
Auf der oberen Seite dieses Komposthaufens PK beträgt die Schicht des kohlensäurehaltigen Komposts KPK 15 bis 35 cm, vorzugsweise 20 bis 30 cm, und 5 bis 10 cm auf den Seitenflächen.
ETAPPE 1 D
Man lässt den Kompost PK seine Fermentation unter der Abdeckung und mit der Synergie der abdeckenden Schicht der KPK weiterführen bis man ein Entweichen von Gasen oben auf dem oberen Teil der Anlage wahrnimmt.
ETAPPE 1E ERNTE
Man vollzieht dann die Ernte von:
der Gesamtheit der oberen und seitlichen Schichten des kohlensäurehaltigen Pflanzenkomplexes KPK (Volumen des Komplexes d1) UND eines Volumens d2 des Komposts PK, der sich zwischen den Schichten KPK und PK befindet.
Bei einer Höhe der Kompostmasse PK von 60-80 cm und einer Höhe der oberen Schicht des KPK von 15-30 cm, vorzugsweise von 20-30 cm, ist die Höhe des Volumens d2 der Kompostmasse PK, die unter der Schnittstelle PK/KPK entnommen wird, 60 bis 80 cm, **Kompromiss** zwischen der Notwendigkeit, über ein möglichst grosses Volumen an d2 zu verfügen, aber nicht zu gross, damit kein Kompost d2 entnommen wird, der nicht ausreichend aktiviert ist. Diese Aktivität kann vor allem durch eine Temperaturmessung und/oder eine Probenahme eingeschätzt werden, um eine Beziehung zwischen der Temperatur und der Bioaktivität festzustellen. Die Bioaktivität wird vor allem durch die Analyse der Gase CO₂ oder NH3 festgestellt.
ETAPPE 1 F Siebdurchgang S
Der geerntete "Dotierte Pflanzenkomplex" DPK, das heisst das Volumen d1 (DPK) und d2 (Kompost PK) wird durch ein Sieb S passiert, das die Partikeln von einer Grösse zwischen ungefähr 1 und 10 mm zurückhält, was ein erstes Produkt von "aktivierten groben Partikeln" bildet oder "GP" und die Partikeln von einer weit unter 1mm liegenden Grösse durchgehen lässt, die hier als "feine aktivierte" Partikeln bezeichnet werden oder "FA", die den dotierten Pflanzenkomplex DPK bilden.
**OPTION 2**
**Überdotierter Pflanzenkomplex (ÜDPK)**
Es werden genau dieselben Etappen wie in der Option 2 ausgeführt, abgesehen davon, dass nach der Abdeckung des Komposts PK durch eine Schicht von kohlensäurehaltigem Pflanzenkomplex KPK alles durch eine dritte Schicht aus groben aktivierten Partikeln GP abgedeckt wird, die vom Sieb in der obenstehenden Option 1 zurückgehalten wurden.
Man nimmt d1 von DPK und d2 von PK, wie in der Option 1, heraus, dann die Schicht GP (generell 40-50-60 cm am oberen Teil), das Verhältnis zwischen der Gesamtheit des Geernteten (d1 + d2 + GA) / nicht benutzter Rest des PK beträgt 50/50 des Volumens.
Nach der Siebung erhält man den "Überdotierten Pflanzenkomplex" ÜDPK.

2. Verfahren nach Anspruch 1, **dadurch gekennzeichnet, dass** man eine Verdünnung vornimmt
**Verdünnung mit einem "neutralen" oder "inerten" Träger**
Der reine Komplex DPK oder ÜDPK *(ohne Unterschied)* gemäss der Erfindung wird mit einem oder mehreren festen, inerten oder neutralen Trägern verdünnt, wie Karbonatsand oder Kieselerde, oder andere Produkte wie die Puzzolane.
Die Verdünnung kann in irgendeinem Verhältnis vorgenommen werden, vor allem in einem Verhältnis DPK oder ÜDPK rein/Sand (Bruttogewicht) von 30/70, 20/80, und sogar 10/90, 5/95, 1/99.
**Verdünnung mit einem Träger des Typs "organische Stoffe" (oder solche organischen Stoffe enthaltend)**
Die Verdünnung kann mit Produkten vollzogen werden, die einen Prozentsatz an organischen Stoffen besitzen wie Rapskuchen, Olivenfruchtfleisch, Kakaoschoten.

3. Produkte DPK und ÜDPK, **dadurch gekennzeichnet, dass** sie durch das Verfahren der vorausgehenden Ansprüche erhalten worden sind.

4. Produkte gemäss Anspruch3, die durch mindestens eines der folgenden Merkmale gekennzeichnet sind ;
- die Gase NH3, CO2, auch H2 sind "blockiert" und fixiert, was eine Kohlenstoff- und Stickstoffsenke darstellt;
- die Konzentration von Huminsäurekernen und Huminsäuren beträgt 200 bis 600% (vor allem 300-500%) mehr als die erwartete Konzentration
- die sehr grosse Konzentration und die sehr grosse Vielfalt an Mikroorganismen (Bakterien, Pilze, Schimmel, ...), die ihrerseits Huminsäuren herstellen
- eine aussergewöhnliche Konzentration von Mykorrhizen, ein Hemicellulosegehalt in den Komplexen DPK oder ÜDPK nach 6 bis 8 Monaten von Interaktionen, der fast doppelt so hoch ist wie in einem gewöhnlichen Kompost.

5. Verwendung wenigstens eines Produkts DPK oder ÜDPK gemäss den Ansprüchen 3 oder 4, oder wie in den Ansprüchen 1 oder 2 beschrieben,
rein oder verdünnt, in einer Biogasanlage, um Methan oder Biogas herzustellen.

6. Verwendung gemäss dem Anspruch 5, das heisst für eine Biogasanlage von 3000 m³, die organische Abfälle benutzt und einen täglichen Zusatz von 60 t dieser organischen Abfälle, werden diesem täglichen Zusatz von 60 t 600 kg DPK oder ÜDPK erfindungsgemäss rein hinzugefügt und vermischt, in den angegebenen Anteilen mit Sand und vorzugsweise mit Karbonatsand verdünnt, anteilmässig zwischen 35 bis 60% des Gewichts, vorzugsweise zwischen 45 bis 54% des Gewichts bezüglich des reinen Produkts DPK oder ÜDPK.

7. Verwendung gemäss den Ansprüchen 5 oder 6, **dadurch gekennzeichnet, dass**
man im unteren Teil des Reaktors das sogenannte "**Gärgut**" findet.
Ich, die Unterzeichnete, Brigitte Jeannin, vereidigte Übersetzerin beim Berufungsgericht RIOM, bescheinige, dass die obenstehende Übersetzung mit dem Original in französischer Sprache übereinstimmt.
NE VARIETUR,Nr. 250/2019

## Claims

1. Method of preparation of doped or over-doped Plant Complexes (DPC or ODPC) "by compost coverage" **characterised in that** it includes the following steps:
***STEP A***
***Preparation of the activated plant complexes (PC)***
Step A1
A certain number, between 6 and 20 types, preferably 10 to 15 types, of plant matter, so-called "plant complexes" or PC are harvested, each PC being composed *of a single plant or special plant matter,* plants or *plant* matter, which comprise at least
i:
- nettles,
- oak leaves, and
- fungi, moulds and spores present in the soil, develop e.g. under fallen leaves, or in the
soil, on the roots,
- and optionally other plants or *plant* matter depending on local availabilities
and each plant or plant matter is spread separately over a large surface area, e.g. one ha (hectare), or possibly half
buried in a trench T laid out in the soil (S).
STEP2
1 *First fraction of these plants or plant matter:*
Each plant or plant matter PC1 of this first fraction is spread over a large surface area, e.g. one ha, *half* buried in a trench T and *half* (above ground", in particular nettles or oak leaves and possibly other plants;
2 *Second fraction of these plants or plant matter:*
Each plant or plant matter PC2 of this second fraction is spread over a large surface area e.g. one ha, totally "above ground", in particular leaves, fungi and other plants;
3 Each of these spreads of plant or plant matter is
covered by a thickness of UN-TREATED straw S, in particular wheat or spelt;
4 Each "spread" preferably has a thickness on the order of 80 cm to 1 m, and the thickness of the straw S layer is
20 on the order of 20 to 40 cm;
STEP3
All of the spreads are left to degrade for 18 to 24 months, preferably 2 years, for half-buried spreads of the "first half-buried fraction" and for 2 to 8 months, preferably 3 to 6 months for totally above-ground spreads of the "second fraction";
STEP A4
6 Harvest:
At the end of the above periods, ALL of the degraded straw and degraded plants or plant matter and their degradation products, including fungi, which form as many
"plant complexes" PC as the piles of spread, are harvested and these harvests of plant complexes, i.e. as many PCi as "straw covered spreads", are grouped together and mixed, which forms the **"Plant Complex" PC** (global).
***STEP B***
***Preparation of highly activated "carbonated plant (CPC) complexes"***
The (activated) plant complex" PC prepared in step B is
- B1 dried until the concentration of dry matter
is of about 60 to 80% by weight, and is then
- B2 mixed with a **"carbonate material"** which is any material containing a high or very high proportion of carbonate, preferably **of calcium carbonate** in a form with a coarse or very coarse particle size distribution
in particular CCC or crushed natural calcium carbonates, e.g. marble, calcite, Aragonite and with a particle size distribution in the region of 100 to 300
microns, or even coarser, and preferably with a heterogeneous or very heterogeneous particle size distribution, the proportion of "carbonate material" and in particular calcium carbonate being from 70 to 95% by weight of dry carbonate material (the amount of dry matter in a carbonate is on the order of 98% by weight), preferably 80 to 90%, the supplement up to 100% being the global plant complex at 60-80% by weight of dry matter, i.e. 5 to 30%, preferably 10 to 20%, of raw plant complex.
***METHOD OF PREPARATION of DOPED OR OVER-DOPED PLANT COMPLEXES***
**OPTION 1**
**Doped plant complex (DPC)**
Step 1 A
A compost with a very high proportion of straw or equivalent plat matter, most preferably a straw, and
an organic matter in particular a compost of straw and horse dung with preferably 90% by weight of straw and 10% by weight of horse dung and which preferentially contains 20 to 40% of dry matter by weight, the balance being water. A CPC is laid on a different PC, e.g.
a **plant** compost on **cattle or horse** (equine) manure or a **plant** compost on the manure of other domestic animals
the proportion of animal matter in the PC finally always being less than 1% by weight in the materials given to micro-organisms to develop.
STEP 1 B
This compost is allowed to ferment in aerobic conditions for 3 to 6 days, which produces the fermented compost or the fermenting PC.
STEP 1C
The PC compost is laid on the ground in the form of a pile, preferably generally trapezoidal in cross-section, and all its faces, in particular the upper face, are covered with a layer of the Carbonated Plant Complex CPC obtained as above.
The height of the pile of PC compost is on the order of 1.70 to 1.90 m.
On the upper face of this pile of PC compost, the layer of carbonated plant complex CPC is on the order of 15 to 35, preferably 20 to 30 cm, and 5 to 10 cm on the side faces.
STEP 1 D
The PC compost is allowed to continue its fermentation under the cover of, and in synergy with, the covering layer of CPC, just until a release of gas is perceived at the top of the assembly.
STEP 1 E HARVEST
The harvesting is then performed, of:
all of the upper and side layers of the carbonated plant complex CPC (volume of compost d1) and a volume d2 of PC compost situated under the interface between CPC and PC.
For a height of the bulk of the PC compost of 60-80 cm and a height of the upper layer of CPC of 15-30, preferably 20-30 cm, the height of the volume d2 of the bulk of PC compost sampled under the PC/CPC interface, is on the order of 60 to 80 cm, **included** between the
necessity of laying as large a recovered volume d2 as possible, but not too large so as not to gather a too-little activated compost d2, said activity being recognised in particular by a measurement of temperature and/or sampling to establish the relation between the temperature and
the bioactivity, the bioactivity being recognised in particular by the analysis of gases such as analyse CO2 or NH3.
STEP 1 F Passage to riddle or sieve SI
The "Doped Plant complex" DPC harvested, i.e. the volume d1 (DPC) and d2 (PC compost) is passed through a riddle or sieve SI which retains particles of sizes between about 1 and 10 mm, which form a first product of "activated coarse particles" or "AC" and allows particles substantially smaller in size than 1 mm, hereafter called "activated fines" or "AF" which form the Doped Plant Complex DPC.
**OPTION 2**
**Over-doped Plant complex (ODPC)**
Strictly the same steps as in option 1 are implemented, except **in that** after having covered the PC compost by a layer of CPC (carbonated plant complex), the whole is covered by a
third layer composed of activated coarse particles (AC) obtained from that retained by the sieve in option 1 above.
D1 is recovered from CPC and d2 from PC as in option 1, plus the AC layer (in general of 40 - 50 - 60 cm at the top) GA, the ratio between all that is harvested (d1 + d2 + AC) / the unused retained part of PC, then being on the order of 50/50 by volume.
After riddling, the "Over-doped Plant Complex" ODPC is obtained

2. Process according to claim 1 **characterised in that** the process has a dilution
**Dilution with a "neutral" or "inert" support**
Indifferently, the pure DPC or ODPC according to the invention is diluted with one or more inert, or neutral, solid supports, such as carbonate or silica sand, or other products such as pozzolans.
The dilution can be achieved with almost any proportion, in particular with a pure DPC or ODPC / sand ratio (by gross weight) of 30/70, 20/80, or even 10/90, 5/95, 1/99.
**Dilution with an "organic material" type support (or containing such organic material)** The dilution can be achieved with products containing a % of organic material such as rapeseed cake, olive pulp, cocoa pods.

3. DPC and ODPC products **characterised in that** said have been obtained by the process according to some of the preceding claims.

4. Products according to claim 3 **characterised by** at least one of the following characteristics;
the gases NH3, C02, and H2 have been "blocked" and fixed, which represents a carbon and nitrogen well;
the concentration of humic acid kernels and humic acids is 300 to 600% (in particular 300-500%) greater than the expected concentration
the very high concentration and the very great diversity of micro-organisms (bacteria, fungi, moulds, etc.) for producing humic acid in their turn.
an exceptional concentration in mycorrhizae, a concentration of heli-cellulose in the DPC or ODPC complex of about double in terms of 6 to 8 months interactions compared to normal compost.

5. Use of at least one DPC or ODPC product according to claims 3 or 4 or such as described in claim 1 or 2, pure or diluted, in a biogas plant for producing methane or biogas.

6. Use, according to claim 5 **characterised in that**, for a biogas plant of 3000m3 using organic wastes, and a
daily supplement of 60 t of said organic wastes, a mixture is added to said 60 t of a daily supplement of 600 kg of DPC or ODPC according to the invention which has been diluted in the proportions indicated with sand and very preferably with carbonate sand, in a proportion of 35 to 60% by weight brought to CaO, preferably of 45 to
54% by weight with respect to the pure DPC or ODPC product.

7. Use according to claim 5 or 6 **characterised in that**
a so-called **"digestate"** is recovered from the bottom of the reactor.
